# EUROPEAN PATENT SPECIFICATION

(11) **EP 0 794 806 B1**
(45) Date of publication and mention of the grant of the patent: **20.01.1999**
(21) Application number: 94929042.3
(22) Date of filing: 24.08.1994
(51) Int. Cl.: A61M 16/00

(54) **RESPIRATION MASK**
BEATMUNGSMASKE
MASQUE POUR LA RESPIRATION ARTIFICIELLE

(43) Date of publication of application: 17.09.1997
(73) Proprietor: I.M.G. B.V., 5438 Gassel (NL)
(72) Inventor: VAN DE VORLE-HOUBEN, Elisabeth, Johanna, Jacoba, NL-5438 NH Gassel (NL)
(74) Representative: Smulders, Theodorus A.H.J., Ir.
(86) International application number: NL9400203
(87) International publication number: WO9605880

(56) References cited:
- EP-A- 0 352 866
- EP-A- 0 442 008
- DE-U- 8 907 096
- US-A- 3 626 936
- US-A- 4 819 627
- US-A- 5 119 809

## Description

The invention relates to a respiration mask consisting of a supple synthetic foil having a pass portion that is placeable over a mouth and/or nose of for instance a patient, which pass portion is provided with an opening covered by an air-permeable material, means being incorporated for obstructing the air permeability of the pass portion in the exhaling direction of the patient while the air permeability in the inhaling direction of the patient is at least substantially unobstructed. Such a mask is known from European patent specification 0 352 866.

The known respiration mask consists of a plastic foil made from supple synthetic material, incorporating a pass portion wherein an air-permeable fabric is placed. During use, this pass portion can be placed over the mouth or nose of the patient, the foil being draped over at least a large part of the rest of the patient's face. Subsequently, the helper puts his mouth on the outside of the pass portion and blows air through the air-permeable fabric into the patient's mouth. Because the respiration mask, and in particular the air-permeable fabric, is disposed between the helper and the patient, and the pass portion passes air in the direction of the patient more easily than in opposite direction, direct contact between the patient and the helper is avoided. This advantageously reduces the chances of infection and fouling when aid is being given.

However, the known respiration mask has as a drawback that when the mask, and in particular the air-permeable fabric, becomes wet, the protection offered by the respiration mask is insufficient. When a patient is given respiratory resuscitation, pressure is exerted on the air-permeable fabric to enable supplying sufficient air to the patient. When pressure is exerted on the air-permeable material if it is for instance soaked with blood or saliva or a like moisture, this moisture is pressed through the air-permeable material. This as yet brings about contact and hence danger of contamination when aid is being given. Moreover, the air permeability of the material also rapidly becomes insufficient in the direction of the patient when much moisture is absorbed and pressure is exerted on the pass portion.

Hence, the object of the invention is to provide a respiration mask of the above-mentioned type wherein the drawbacks mentioned are avoided and the advantages are retained. To that end, the respiration mask according to the invention is characterized in that at the pass portion spacer means are provided which, during use, keep the air-permeable material at a distance from at least one of the mouths or nose involved in the respiratory resuscitation and provide a distribution of the pressure exerted during the respiratory resuscitation, the air-permeable material together with the spacer means forming a fluid barrier at least in exhaling direction of the patient.

By providing the spacer means at the pass portion, a distance is created between at least one of the mouths or nose involved in the respiratory resuscitation and the air-permeable material. Moreover, the pressure exerted on the pass portion during the respiratory resuscitation by at least the helper is favorably distributed over the respiration mask. This on the one hand prevents moisture from passing the air-permeable material all the same, due to the compression of the pass portion, and on the other hand the distance created offers the air exhaled by the patient the possibility of escaping in a way other than in the direction of the helper's mouth. Further, by virtue of the spacer means a psychological advantage is achieved in that the helper observes a clearer barrier between himself and the patient, as a result of which the helper will sooner be prepared to actually render assistance. After all, he will feel less fear of contamination and less physical aversion.

In further elaboration, the invention is characterized in that during use the spacer means at least partly abut against the foil, spaced from the opening. This causes the pressure exerted during the respiratory resuscitation to be at least largely transmitted to a part of the respiration mask that is incapable of absorbing moisture and that can be brought into direct contact with a part of the face of the patient. As a result, the pressure on the pass portion can almost entirely be removed.

Further, the use of the known respiration mask involves the risk that due to moisture, such as blood, saliva and perspiration, accumulating between the respiration mask and the patient's face, the foil will adhere to the patient's skin, directly around the pass portion. This will prevent the air exhaled by the patient from flowing away in a way other than via the pass portion, in the direction of the helper. This further increases the chances of contamination.

To avoid this risk, in an advantageous embodiment according the the invention, the respiration mask is characterized in that the spacer means are provided on the side of the respiration mask facing the patient during use, the spacer means being preferably substantially impermeable to fluid and in particular to saliva and blood but permeable to air, at least in exhaling direction of the patient.

Because the spacer means are provided on the side facing the patient during use, the foil is kept at a distance from the patient's face at least directly around the pass portion, allowing air to flow away in a simple manner between the foil and the patient's skin. Because the spacer means are substantially fluid-tight in exhaling direction, the air-permeable material is moreover prevented from becoming moist through the patient, as a result of which a complete fluid barrier is obtained, while the air is can flow in an unobstructed manner in the direction of the patient but not back in the direction of the helper.

In a further advantageous embodiment, the respiration mask is characterized in that the spacer means are formed from a compressible, non-absorbing synthetic fiber material that is open at least toward a part of the sides transverse to the breathing direction of the patient, such as for instance a strip of ventilation band having an open network structure and at least partly covering the pass portion. This embodiment can be manufactured in a simple and relatively cheap manner, while the fiber material simply enables the air exhaled by the patient, possibly reaching the fiber material, to flow away to the sides.

In a preferred embodiment, the respiration mask according to the invention is characterized in that the foil is substantially clearly transparent, with instructions for use preferably provided on the foil. Because the foil, draped over the patient's face, is clearly transparent, the patient's reactions, such as color changes in the skin and pupil reactions, can easily be detected by the helper. Because the instructions for use are provided on the foil, for instance by means of a printing technique, they are always present at the mask, as a result of which the respiration mask and the associated respiration technique will also be properly applied in a situation of panic, which often occurs when someone suffers from lack of breath. Moreover, this renders the respiration mask particularly suitable for use during training.

To explain the invention, a number of exemplary embodiments of a respiration mask according to the invention will be described hereinafter, with reference to the accompanying drawings. In these drawings:
Fig. 1 is a front view of a respiration mask provided over a face;
Fig. 2 is a side elevation of the respiration mask according to Fig. 1;
Fig. 3 is a portion of a cross-section taken on the line III-III in Fig. 1 to an enlarged scale;
Fig. 4 is a front view of a respiration mask in a first alternative embodiment;
Fig. 5 shows a cross-section of the pass portion of a respiration mask taken on the line V-V in Fig. 4; and
Fig. 6 shows a cross-section of the pass portion of a respiration mask in a second alternative embodiment.

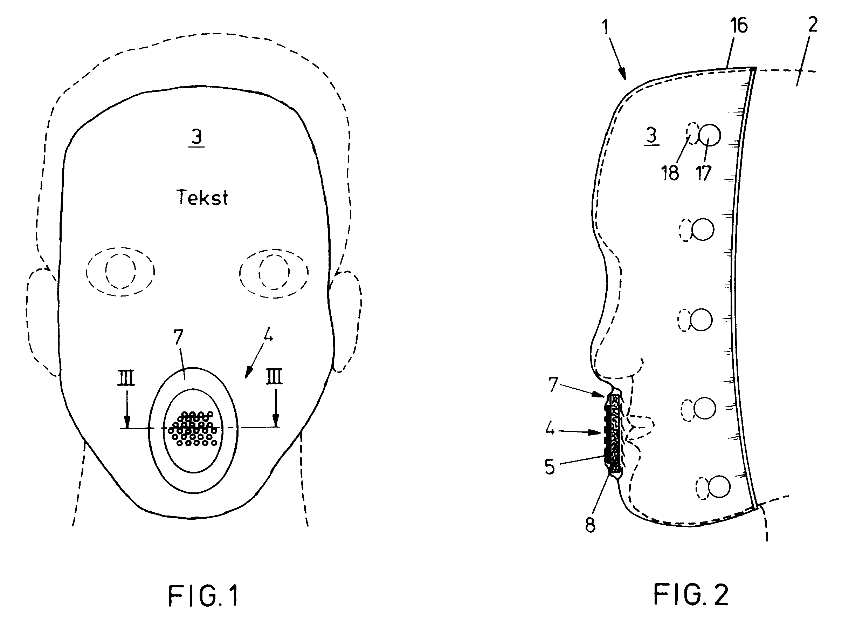

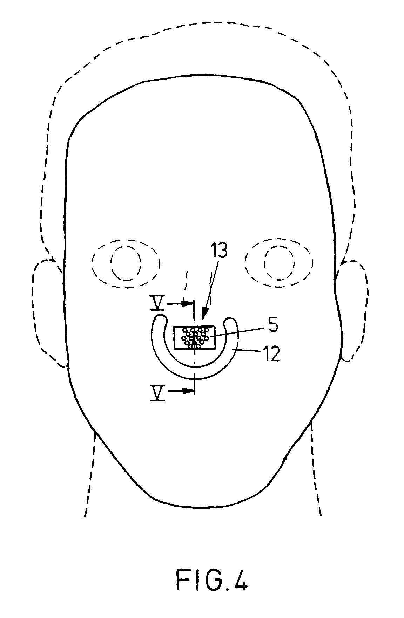

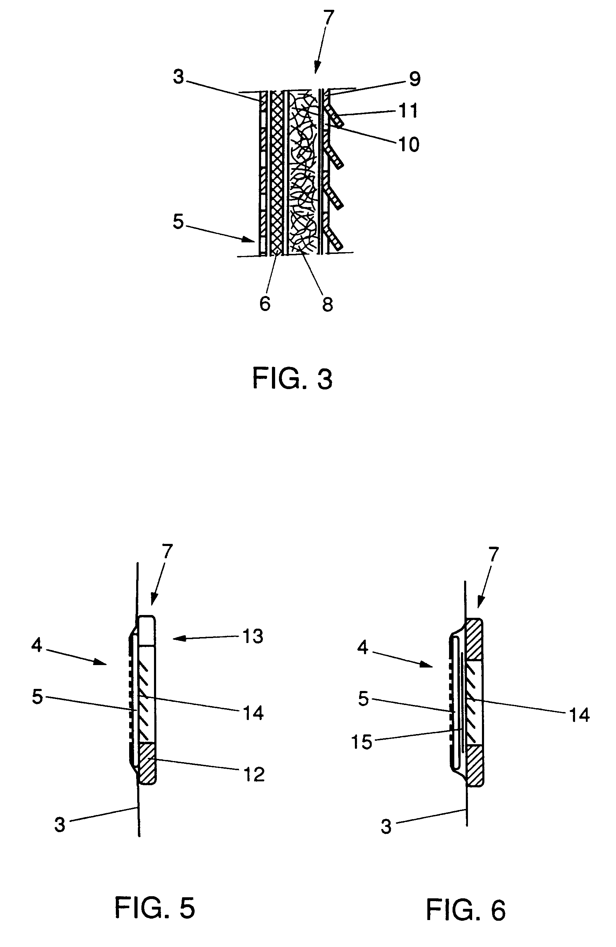

Figs 1 and 2 show a respiration mask 1 provided over a patient's head 2 shown in broken lines. The respiration mask consists of a supple, clearly transparent synthetic foil 3, incorporating a pass portion 4 which in the position shown in Figs 1 and 2 covers the mouth of the head 2. Fig. 3 shows to an enlarged scale a cross-section of the pass portion 4.

In the pass portion a number of first openings 5 are incorporated, which openings 5 are covered, on the inside of the respiration mask 1 facing the head, by a gauze-shaped covering 6. This covering 6 has a good air permeability of its own and is at least in non-compressed form moisture-proof, and guarantees a physical barrier between the mouth or nose of the patient on the one hand and the mouth of the helper on the other. The covering can for instance consist of synthetic material, muslin, fine muslin, fiber cloth or the like.

On the side of the pass portion 4 facing the head 2 of the patient, spacer means 7 are provided, which keep the gauze-shaped covering 6 at a distance from the patient's mouth. In the embodiment shown in Fig. 1, these spacer means 7 consist of a strip 8 of non-absorbing synthetic fiber material having an open network structure and to be referred to as ventilation band, which strip covers the gauze-shaped covering 5 completely. The strip of ventilation band 8 is slightly deformable to prevent damage to the respiration mask 1 on the one hand and to the mouth or nose of the patient on the other. Preferably, the strip 8 abuts against the foil 3 along its entire longitudinal edge so that when pressure is being applied to the respiration mask by the mouth of the helper, this pressure is substantially transmitted to the skin of the patient around his lips or nose, and not to the gauze-shaped material.

On the side facing the patient's mouth, the strip 8 is covered with a layer of foil 9 provided with second openings 10. Each second opening 10 comprises a valve part 11, provided for swivelling movement, which in the first position shown in Fig. 3 leaves the opening 10 clear as the valve part has been swivelled away in the direction of the patient. However, in a second position, wherein the valve part 11 is substantially parallel to the foil layer 9, in or over the opening 10, the opening 10 is entirely covered by the valve part 11, preventing the backflow of air from the patient through the pass portion 4. The valve parts 11 are moved under the influence of air flowing through the pass portion in the direction of the patient and by air exhaled by the patient.

Apart from the fact that they provide a proper pressure distribution and pressure transmission between the patient and the helper, and hence a proper protection against infection, the spacer means 7 have the advantage that the foil 3 is at least partly kept at a distance from the patient's face thereby, also if the facial skin of the patient is moist, for instance with blood, saliva, perspiration, water or the like. Hence, a space is kept open between the foil 3 and the patient's skin along which the air exhaled by the patient can escape. In this manner, this exhaled air is prevented from being forced back through the gauze-shaped material in the direction of the helper or is trapped under the respiration mask.

Along its outer edge, the respiration mask 1 is provided with an elastic edge 16 with which the foil 3 can be secured over the patient's face. Near the elastic edge 16 a number of third openings 17 are provided in the foil, each third opening 17 comprising, similarly to the first openings 10, second valve parts 18 capable of swivelling outwards. Air exhaled by the patient is capable of flowing along the pass portion, possibly via the spacer means, between the foil 3 and the patient's face and of being discharged outside via the third openings 17. The second valve parts 18 substantially prevent air from outside from flowing to the patient in a manner other than via the pass portion. Because the respiration mask 1 properly fits around the head 2 with the elastic edge 16, it need not be held by the helper, so that both his hands are free during the respiratory resuscitation of the patient. Moreover, this also enables the respiration mask to be utilized as hyperventilation mask, for controlling and regulating respiration.

In the embodiments shown in the drawings, the foil 3 is clearly transparent, with instructions for use printed on the foil. Because the foil 3 is clearly transparent, the patient's face can continuously be observed. Accordingly, changes in for instance the complexion and pupil reactions, or, by contrast, the absence thereof, can well be perceived. This permits the respiration mask to be used for all kinds of patients, also for patients who cannot be expected to show clear pupil reactions due to for instance the use of medicins or drugs. By printing instructions for use on the foil 3 it is ensured in a simple manner that they are always present when the respiration mask 1 is used, also when used repeatedly.

One of the sides, and preferably the inside of the pass portion, is marked through coloring (for instance red), so that it is always clear which side of the pass portion should be turned toward the patient. As a matter of fact, another form of coding is also possible, such as for instance lettering.

Figs 4 and 5 show an alternative embodiment of the pass portion 4 and the spacer means 7. In this embodiment, the spacer means 7 are formed by a ring segment 12 substantially disposed around the gauze-shaped material 5 and interrupted at the upper side thereof. The interrupted part 13 is shaped so that the ring segment 12 can be slid along the patient's nose, permitting, in addition to mouth-to-mouth resuscitation, mouth-to-nose resuscitation. The gauze-shaped material 5 has its inside covered with a foil layer 14 provided, in the manner described hereinabove, with second openings 10 and associated valve parts 11. The ring segment is secured to the foil layer 14 and/or the foil 3 on the side facing the patient.

The ring segment 12 may be manufactured from the above-described fiber material or a like air-permeable material, but may also be manufactured from tight plastic, rubber or a like material.

Fig. 6 shows a further alternative embodiment of the pass portion 4 of a respiration mask, which, as far as its construction is concerned, substantially corresponds to the pass portion as described with reference to Fig. 5, but which incorporates a hydrophobic, air-permeable diaphragm 15 between the foil layer 14 and the gauze-shaped material 5. This diaphragm suitably prevents the gauze-shaped material 5 from becoming moist under the influence of the patient.

Prior to use, the respiration mask is preferably sterilely packed in a partially transparent package, with instructions for use, separately included in the package, being visible from the outside of the package. This enables the helper to study the instructions before taking the respiration mask 1 out of the package, so that a better aid can be given to a patient and the respiration mask, still packed, can already be used for training purposes.

In the drawings, the respiration mask 1 shown is in each case provided over the face of a patient in need of help. However, as the occasion arises, it offers advantages if the helper puts the mask over his own face, for which purpose the respiration mask 1 is turned inside out so that the side provided with the second openings 10 and valve parts 11 always faces the patient. In particular in the case of patients whose faces lie open, for instance with cuts or burns, or patients who might panick when regaining consciousness, a sealing foil 3 over the face will, after all, not always be advantageous.

The invention is by no means limited to the embodiments as described. Many variants are possible. For instance, the flow-back of air from the patient in the direction of the helper can be prevented by incorporating, on the side of the pass portion facing the patient, two foil layers lying directly on top of each other, both having a number of openings that are staggered relative to each other. When air is blown in the direction of the patient, the foil layers will be pressed apart, permitting air to pass the openings in the two foil layers. In opposite direction, the material of the outer foil layer (located closest to the patient) extending between the openings will be pressed over the openings of the other foil layer so that they are closed and the flow-through of air is hence prevented. The respiration mask is not only suitable for use with patients, but can moreover very suitably be used for training on dummies and the like. Through a suitable folding manner, the respiration mask can be stored small and compact, enabling it to be readily carried along. Moreover, a codicil and other personal data of the bearer can for instance be enclosed. These and other adaptations are considered to be within the purview of the invention as claimed.

## Claims

1. A respiration mask (1) consisting of a supple synthetic foil (3), comprising a pass portion (4) placeable over a mouth and/or nose of for instance a patient, said pass portion (4) being provided with at least one opening (5) covered by an air-permeable material (6), means being incorporated for obstructing the air permeability of the pass portion in the exhaling direction of the patient, while the air permeability in the inhaling direction of the patient is at least substantially unobstructed, characterized in that at the pass portion spacer means (7) are provided which, during use, keep the air-permeable material (6) at a distance from at least one of the mouths or nose involved in the respiratory resuscitation and provide a distribution of the pressure exerted during the respiratory resuscitation, the air-permeable material together with the spacer means forming a fluid barrier at least in exhaling direction of the patient.

2. A respiration mask according to claim 1, characterized in that the spacer means (7), during use, at leapt partly abut against the foil (3), spaced from the opening (5).

3. A respiration mask according to claim 1 or 2, characterized in that the spacer means (7) are provided on the side of the mask facing the patient during use.

4. A respiration mask according to any one of the preceding claims, characterized in that the spacer means (7) are substantially impermeable to fluid and in particular at least substantially impermeable to saliva and blood but permeable to air, at least in exhaling direction of the patient.

5. A respiration mask according to any one of the preceding claims, characterized in that at least one of the sides of at least the pass portion is marked, for instance by text or color, so that it is clear to the user which side should face the patient during use.

6. A respiration mask according to any one of the preceding claims, characterized in that the spacer means (7) are formed from a flexible, non-absorbing synthetic fiber material that is open at least toward a part of the sides transverse to the breathing direction of the patient.

7. A respiration mask according to claim 6, characterized in that the spacer means (7) comprise a strip (8) of ventilation band having an open network structure and at least partly covering the pass portion.

8. A respiration mask according to any one of the preceding claims, characterized in that the spacer mean (7) comprise a ring (12) extending along at least a part of the circumference of the opening.

9. A respiration mask according to any one of the preceding claims, characterized in that the spacer means (7) comprise a hydrophobic, air-permeable diaphragm (15) covering the opening (5).

10. A respiration mask according to any one of the preceding claims, characterized in that the means obstructing the air permeability comprise a foil layer (14) having first openings (5) provided therein, each first opening having a first valve part (11) provided for swivelling movement and preferably formed from the foil, covering the relevant opening in a first position and leaving the relevant opening clear in a second position swivelled in the direction of the patient, each valve part capable of swivelling under the influence of air displacement.

11. A respiration mask according to any one of the preceding claims, characterized in that the means obstructing the air permeability comprise a number of passages with decreasing cross-sections, viewed in the direction of respiratory resuscitation.

12. A respiration mask according to any one of the preceding claims, characterized in that the mask comprises an elastic circumferential edge (16) capable of at least partly enveloping the patient's head during use, with a number of second openings (10) provided near the circumferential edge (16), spaced from the pass portion.

13. A respiration mask according to claim 12, characterized in that each second opening (10) comprises a second valve part (18), provided for swivelling movement and preferably formed from the foil, covering the relevant opening in a first position and leaving the relevant opening clear in a second position swivelled in the direction away from the patient, each valve part being capable of swivelling under the influence of air displacement.

14. A respiration mask according to any one of the preceding claims, characterized in that the air-permeable material is provided with an aromatic substance.

15. A respiration mask according to any one of the preceding claims, characterized in that the foil is substantially clearly transparent.

16. A respiration mask according to any one of the preceding claims, characterized in that instructions for use are provided on the foil.

17. A respiration mask according to any one of the preceding claims, characterized in that, prior to use, it is sterilely packed in an at least partly transparent package, with instructions for use, separately included in the package, being visible from the outside of the package.

## Patentansprüche

1. Beatmungsmaske (1), die aus einer elastischen, synthetischen Folie (3) hergestellt ist, die einen Durchlaßbereich (4) hat, der über dem Mund und/oder der Nase von zum Beispiel einem Patienten angeordnet werden kann, wobei der Durchlaßbereich (4) mit zumindest einer Öffnung (5) versehen ist, die durch ein luftdurchlässiges Material (6) überdeckt ist, und wobei Einrichtungen vorgesehen sind, um die Luftdurchlässigkeit des Durchlaßbereiches in der Ausatmungsrichtung des Patienten zu behindern, während die Luftdurchlässigkeit in der Einatmungsrichtung des Patienten zumindest im wesentlichen unbehindert ist, dadurch gekennzeichnet, daß an dem Durchlaßbereich Abstandseinrichtungen (7) vorgesehen sind, durch die das luftdurchlässige Material (6) während der Benutzung entweder von dem an der Beatmungs-Reanimation beteiligten Mund oder Nase beabstandet gehalten ist und durch die eine Ausbreitung des Drucks bewirkt wird, der während der Beatmungs- Reanimation aufgebracht wird, wobei das luftdurchlässige Material zusammen mit den Abstandseinrichtungen zumindest in der Ausatmungsrichtung des Patienten eine Fluidsperre bildet.

2. Beatmungsmaske nach Anspruch 1, dadurch gekennzeichnet, daß die Abstandseinrichtungen (7) während der Benutzung zumindest teilweise gegen die Folie (3) anliegen, und zwar von der Öffnung (5) beabstandet.

3. Beatmungsmaske nach Anspruch 1 oder 2, dadurch gekennzeichnet, daß die Abstandseinrichtungen (7) an der Seite der Maske vorgesehen sind, die bei der Benutzung dem Patienten zugewandt ist.

4. Beatmungsmaske nach einem dar vorhergehenden Ansprüche, dadurch gekennzeichnet, daß die Abstandseinrichtungen (7) im wesentlichen für Fluide und insbesondere zumindest im wesentlichen für Speichel und Blut undurchlässig sind, aber für Luft durchlässig sind, zumindest in Ausatmungsrichtung des Patienten.

5. Beatmungsmaske nach einem der vorhergehenden Ansprüche, dadurch gekennzeichnet, daß zumindest eine der Seiten von zumindest dem Durchlaßbereich markiert ist, beispielsweise durch Beschriftung oder Farbe, so daß es für den Benutzer offensichtlich ist, welche Seite dem Patienten während der Benutzung zugewandt sein soll.

6. Beatmungsmaske nach einem der vorhergehenden Ansprüche, dadurch gekennzeichnet, daß die Abscandseinrichtungen (7) aus einem elastischen, nicht absorbierenden Synthesefasermaterial hergestellt sind, das zumindest in Richtung eines Bereiches der Seiten schräg zu der Atmungsrichtung des Patienten offen ist.

7. Beatmungsmaske nach Anspruch 6, dadurch gekennzeichnet, daß die Abstandseinrichtungen (7) einen Streifen (8) aus Ventilationsband enthalten, das ein offene Netzwerkstruktur hat und zumindest teilweise den Durchlaßbereich überdeckt.

8. Beatmungsmaske nach einem der vorhergehenden Ansprüche, dadurch gekennzeichnet, daß die Abstandseinrichtungen (7) einen Ring (12) enthalten, der sich zumindest entlang eines Bereiches des Umfangs der Öffnung erstreckt.

9. Beatmungsmaske nach einem der vorhergehenden Ansprüche, dadurch gekennzeichnet, daß die Abstandseinrichtungen (7) eine hydrophobe, luftdurchlässige Membran (15) enthalten, die die Öffnung (5) überdeckt.

10. Beatmungsmaske nach einem der vorhergehenden Ansprüche, dadurch gekennzeichnet, daß die Einrichtungen, durch die die Luftdurchlässigkeit behindert wird, eine Folienschicht (14) enthalten, die zumindest erste Öffnungen (5) hat, die darin vorgesehen sind, wobei jede erste Öffnung ein erstes Ventilteil (11) aufweist, das für eine Schwenkbewegung ausgebildet ist, vorzugsweise aus der Folie besteht, die relevante Öffnung in einer ersten Position verschließt und die relevante Öffnung in einer zweiten Position öffnet, indem es in die Richtung auf den Patienten schwenkt, wobei jedes Ventilteil unter der Wirkung von Luftbewegung hin- und herschwenken kann.

11. Beatmungsmaske nach einem der vorhergehenden Ansprüche, dadurch gekennzeichnet, daß die Einrichtungen, die die Luftdurchläßigkeit behindern, eine Anzahl von Durchgängen enthalten, die, gesehen in der Richtung der Beatmungs-Reanimation, abnehmende Querschnitte haben.

12. Beatmungsmaske nach einem der vorhergehenden Ansprüche, dadurch gekennzeichnet, daß die Maske eine elastische Umfangskante (16) hat, die in der Lage ist, während der Benutzung zumindest teilweise den Kopf des Patienten zu umschließen, wobei nahe der Umfangskante (16) und von dem Durchlaßbereich beabstandet eine Anzahl von zweiten Öffnungen vorgesehen ist.

13. Beatmungsmaske nach Anspruch 12, dadurch gekennzeichnet, daß jede zweite Öffnung (16) ein zweites Ventilteil (18) enthält, das für Schwenkbewegungen vorgesehen ist und vorzugsweise aus der Folie hergestellt ist, die relevante Öffnung in einer ersten Position abdeckt und die relevante Öffnung in einer zweiten Position öffnet, indem es in die Richtung weg von dem Patienten schwenkt, wobei jedes Ventilteil unter Wirkung von Luftbewegung hin- und herschwenken kann.

14. Beatmungsmaske nach einem der vorhergehenden Ansprüche, dadurch gekennzeichnet, daß das luftdurchlässige Material mit einer aromatischen Substanz versehen ist.

15. Beatmungsmaske nach einem der vorhergehenden Ansprüche, dadurch gekennzeichnet, daß die Folie im wesentlichen durchsichtig ist.

16. Beatmungsmaske nach einem der vorhergehenden Ansprüche, dadurch gekennzeichnet, daß die Instruktionen zur Benutzung auf der Folie vorgesehen sind.

17. Beatmungsmaske nach einem der vorhergehenden Ansprüche, dadurch gekennzeichnet, daß sie vor der Benutzung in einer zumindest teilweise transparenten Verpackung steril verpackt ist, wobei die Instruktionen zur Benutzung separat in der Verpackung enthalten und von der Außenseite der Verpackung zu sehen sind.

## Revendications

1. Masque respiratoire (1) constitué d'une feuille synthétique souple (3), comprenant uns partie de passage (4) destinée à être placée sur une bouche et/ou un nez par exemple d'un patient, la partie de passage (4) ayant nu moins une ouverture (5) couverte d'un matériau (6) perméable à l'air, un dispositif étant incorporé afin qu'il obstrue la perméabilité à l'air de la partis de passage dans le sens d'exhalaison du patient alors que la perméabilité à l'air dans le sens d'inhalation du patient est au moins pratiquement non obstruée, caractérisé en ce que, dans la partie de passage, un dispositif (7) d'entretoise est disposé afin que, pendant l'utilisation, il maintienne le matériau perméable à l'air (6) à distance au moins de la bouche ou du nez impliqué dans la ressuscitation respiratoire et assure une distribution de la pression exercée pendant la ressuscitation respiratoire, le matériau perméable à l'air et le dispositif d'entretoise formant une barrière au passage du fluide au moins dans la direction d'exhalaison du patient.

2. Masque respiratoire selon la revendication 1, caractérisé en ce que le dispositif d'entretoise (7), pendant l'utilisation, est au moins partiellement en butée contre la feuille (3) à distance de l'ouverture (5).

3. Masque respiratoire selon la revendication 1 ou 2, caractérisé en ce que la dispositif d'entretoise (7) est placé du côté du masque tourné vers le patient pendant l'utilisation.

4. Masque respiratoire selon l'une quelconque des revendications précédentes, caractérisé an ce que le dispositif d'entretoise (7) est pratiquement imperméable aux fluides et en particulier est au moins pratiquement imperméable à la salive et au sang, mais est perméable a l'air, au moins dans la direction d'exhalaison du patient.

5. Masque respiratoire selon l'une quelconque des revendications précédentes, caractérisé on ce que l'un des côtés au moins de la partie de passage au moins est marqué, par exemple par du texte ou une couleur, afin que l'utilisateur voie clairement quel côté doit être tourné vers le patient pendant l'utilisation.

6. Masque respiratoire salon l'une quelconque des revendications précédentes, caractérisé en ce que le dispositif d'entretoise (7) est formé d'un matériau souple de fibres synthétiques non absorbantes qui est ouvert au moins vers une partie des côtés transversaux à la direction de respiration du patient.

7. Masque respiratoire selon la revendication 6, caractérisé en ce que le dispositif d'entretoise (7) comporte un ruban (8) d'une bande de ventilation ayant une structure de réseau ouvert et recouvrant au moins partiellement la partie de passage.

8. Masque respiratoire selon l'une quelconque des revendications précédentes, caractérisé en ce que le dispositif d'entretoise (7) comporte un anneau (12) qui s'étend le long d'une partie au moins de la circonférence de l'ouverture.

9. Masque respiratoire selon l'une quelconque des revendications précédentes, caractérise en ce que le dispositif d'entretoise (7) comporte un diaphragme hydrophobe perméable à l'air (15) qui recouvre l'ouverture (5).

10. Masque respiratoire selon l'une quelconque des revendications précédentes, caractérisé en ce que le dispositif qui obstrue la perméabilité à l'air comporte une couche d'une feuille (14) ayant des premières ouvertures (5) formées dans la feuille, chaque première ouverture ayant une première partie (11) de valve destinée à présenter un mouvement de pivotement et formée de préférence à partir de la feuille, recouvrant l'ouverture correspondante dans une première position et laissant l'ouverture correspondante dégagée dans une seconde position après pivotement vers le patient, chaque partie de valve pouvant pivoter sous l'influence du déplacement d'air.

11. Masque respiratoire selon l'une quelconque des revendications précédentes, caractérisé en ce que le dispositif qui obstrue la perméabilité à l'air comprend un certain nombre de passages ayant des sections qui diminuent, dans la direction de la ressuscitation respiratoire.

12. Masque respiratoire selon l'une quelconque des revendications précédentes, caractérisé en ce que le masque comporte un bord circonférentiel élastique (16) qui peut envelopper au moins partiellement la tête du patient pendant l'utilisation, un certain noire de secondes ouvertures (10) étant formées près du bord circonférentiel (16) à distance de la partie de passage.

13. Masque respiratoire selon la revendication 12, caractérisé en ce que chaque seconde ouverture (10) comporte une seconde partie de valve (11) destinée à présenter un mouvement de pivotement et formée de préférence à partir de la feuille, recouvrant l'ouverture correspondante dans une première position et dégageant l'ouverture correspondante dans une seconde position après pivotement du côté opposé au patient, chaque partie de valve pouvant pivoter sous l'influence du déplacement d'air.

14. Masque respiratoire selon l'une quelconque des revendications précédentes caractérisé en ce que le matériau perméable a l'air comporte une substance aromatique.

15. Masque respiratoire selon l'une quelconque des revendications précédentes, caractérisé en ce que la feuille est pratiquement transparente.

16. Masque respiratoire selon l'une quelconque des revendications précédentes, caractérisé en ce que des instructions d'utilisation sont placées sur la feuille.

17. Masque respiratoire selon l'une quelconque des revendications précédentes, caractérisé en ce que, avant utilisation, il est conditionné sous forme stérile dans un conditionnement au moins partiellement transparent, avec des instructions d'utilisation, incluses séparément dans le conditionnement et visibles depuis l'extérieur du conditionnement.
